(19) Europäisches Patentamt
European Patent Office
Office européen des brevets

(11) **EP 4 481 336 B1**

(12) **EUROPÄISCHE PATENTSCHRIFT**

(45) Veröffentlichungstag und Bekanntmachung des Hinweises auf die Patenterteilung:
**10.12.2025 Patentblatt 2025/50**

(21) Anmeldenummer: **24175898.6**

(22) Anmeldetag: **15.05.2024**

(51) Internationale Patentklassifikation (IPC):
**G01F 1/667** *(2022.01)* **G01N 29/024** *(2006.01)*
**G01N 33/22** *(2006.01)*

(52) Gemeinsame Patentklassifikation (CPC):
**G01F 1/668; G01F 1/667;** G01N 29/024;
G01N 33/225

(54) **COMPUTERIMPLEMENTIERTES VERFAHREN ZUR ULTRASCHALLDURCHFLUSSMESSUNG, SOWIE VORRICHTUNG ZUR DURCHFÜHRUNG DES VERFAHRENS**

COMPUTER-IMPLEMENTED METHOD FOR ULTRASONIC FLOW MEASUREMENT AND DEVICE FOR CARRYING OUT SAID METHOD

PROCÉDÉ MIS EN OEUVRE PAR ORDINATEUR POUR LA MESURE DE DÉBIT PAR ULTRASONS ET DISPOSITIF POUR LA MISE EN OEUVRE DU PROCÉDÉ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **19.06.2023 DE 102023115959**

(43) Veröffentlichungstag der Anmeldung:
**25.12.2024 Patentblatt 2024/52**

(73) Patentinhaber: **FLEXIM Flexible Industriemesstechnik GmbH**
**12681 Berlin (DE)**

(72) Erfinder:
• **HOHEISEL, Gerald, Dr.**
**12159 Berlin (DE)**
• **Ulbricht, David**
**10119 Berlin (DE)**

(74) Vertreter: **Ellis, Christopher Paul**
**Ollila Law Limited**
**Unit 7 The Courtyard**
**Timothys Bridge Road**
**Stratford upon Avon CV37 9NP (GB)**

(56) Entgegenhaltungen:
**EP-A2- 1 063 525          WO-A1-2020/112731**
**US-A1- 2003 114 992**

• **HOHEISEL G. ET AL: "Fluxus Natural Gas Engine", 10 August 2020 (2020-08-10), pages 1 - 22, XP093216856, Retrieved from the Internet <URL:https://nfogm.no/wp-content/uploads/2022/01/D2_02-Carolina-Stopkoski-FLEXIM.pdf> [retrieved on 20241021]**
• **FARZANEH-GORD MAHMOOD ET AL: "An intelligent approach for calculating natural gas compressibility factor and its application in ultrasonic flow meters", FLOW MEASUREMENT AND INSTRUMENTATION, BUTTERWORTH-HEINEMANN, OXFORD, GB, vol. 76, 4 October 2020 (2020-10-04), XP086386321, ISSN: 0955-5986, [retrieved on 20201004], DOI: 10.1016/J.FLOWMEASINST.2020.101833**

**Beschreibung**

Die Erfindung betrifft ein computerimplementiertes Verfahren zur Ultraschalldurchflussmessung, sowie eine Vorrichtung zur Durchführung des Verfahrens.

In der Anmeldung werden verschiedene Begrifflichkeiten verwendet, welche im Folgenden erläutert werden.

Als Gasbeschaffenheit wird die Beschaffenheit eines Gases bezeichnet. Die Gasbeschaffenheitseigenschaft ist v.a. die Zusammensetzung des Gases aus seinen einzelnen Komponenten. Ein fossiles Erdgas beispielsweise besteht aus vielen Einzelgasen. Als Gasbeschaffenheit wird bei fossilen Erdgasen daher die Zusammensetzung mit typisch bis zu 21 Einzelkomponenten mit Methan als Hauptkomponente bezeichnet.

Synthetische Gasbeschaffenheiten unterscheiden sich von den Gasbeschaffenheiten realer Erdgasmessstellen dadurch, dass die Gasbeschaffenheit durch einen Algorithmus künstlich erzeugt wird. Ausgegangen wird von der Gasbeschaffenheit des Referenzgases. Die Einzelkomponenten werden nun künstlich variiert. Eine einfache Methode, die ein statisches Verhalten simuliert, ist zum Beispiel die Monte Carlo Methode. Aber auch andere Simulationsmethoden sind denkbar.

Unter Normbedingungen werden Referenzbedingungen für Kompressibilität ($Z\_b$), Druck ($P\_b$) und Temperatur ($T\_b$) bezeichnet.

Als Arbeitspunkt werden der an einer Messstelle vorherrschende Druck ($P\_op$) und Temperatur ($T\_op$) bezeichnet. Diese Werte können deutlich verschieden sein zu den Drücken und Temperaturen unter Normbedingungen, beispielsweise in Transportpipelines herrscht ein deutlich höherer Druck als unter Normbedingungen.

Ein Arbeitsbereich definiert typische minimale und maximale Temperaturen ($T\_min/T\_max$) und typische minimale und maximale Drücke ($P\_min/P\_max$) an einer Messstelle.

Als ein Referenzgas wird eine repräsentative Gasbeschaffenheit an der Messstelle bezeichnet, welches dem tatsächlich an der Messstelle vorliegenden Gas entspricht.

Zustandsgrößen sind ein zentraler Begriff aus der Thermodynamik. Die Zustandsgrößen sind die messbaren Eigenschaften eines Systems, welche sich aus Zustandsgleichungen ableiten lassen. Über diese Zustandsgleichungen sind alle Zustandsgrößen miteinander verknüpft. Insgesamt beschreiben alle Zustandsgrößen zusammen den Zustand eines thermodynamischen Systems. Mit ihnen lassen sich in der Verfahrenstechnik das Verhalten von Stoffen beschreiben, z.B. bei der Energieumwandlung oder beim Transport. Zustandsgrößen sind unter anderem Druck (p), Temperatur (T), Volumen (V), Stoffmenge (n), Kompressibilität (z), Brennwert (HV) und/oder Schallgeschwindigkeit (c).

Mit Messstelle wird im Allgemeinen eine ortsfeste oder mobile bauliche Einrichtung bezeichnet, an der bezogen auf einen Messpunkt über eine festgelegte Dauer während einer Messung ein physikalischer und/oder chemischer Parameter aufgezeichnet werden kann.

Ultraschall-Durchflussmesser messen die Geschwindigkeit eines strömenden Mediums (Gas, Flüssigkeit) mit Hilfe akustischer Wellen. Diese Durchflussmesseinrichtung besteht aus zwei Teilen: dem eigentlichen Messaufnehmer (Ultraschallsensor) sowie einem Auswerte- und Speiseteil (Transmitter oder Messumformer). Die akustische Durchflussmessung bietet einige Vorzüge gegenüber anderen Messverfahren. Die Messung ist weitgehend unabhängig von den Eigenschaften der verwendeten Medien wie elektrische Leitfähigkeit, Dichte, Temperatur und Viskosität. Das Fehlen bewegter mechanischer Teile verringert den Wartungsaufwand und ein Druckverlust durch Querschnittsverengung entsteht nicht. Ein großer Messbereich zählt zu den weiteren positiven Eigenschaften dieses Verfahrens. Für die akustische Strömungsmessung mittels Ultraschalls kommen in industriellen Anlagen zwei wesentliche Messprinzipien zum Einsatz: Das Ultraschall-Doppler-Verfahren und Ultraschall-Laufzeit-Verfahren.

Das Laufzeitdifferenzverfahren macht sich die Tatsache zunutze, dass die Ausbreitungsgeschwindigkeit eines Ultraschallschallsignals von der Strömungsgeschwindigkeit des Trägermediums abhängt. Ähnlich einem Schwimmer, der gegen den Strom schwimmt, bewegt sich ein Ultraschallsignal entgegen der Flussrichtung des Mediums langsamer als in Flussrichtung. Beim Laufzeitdifferenzverfahren wird ein Ultraschallimpuls durch das Medium in Strömungsrichtung gesendet, ein zweiter in der entgegengesetzten Richtung. Die Sensoren arbeiten dabei abwechselnd als Sender und Empfänger. Die Laufzeit der sich mit dem Strom ausbreitenden Ultraschallsignale ist kürzer als jene gegen die Strömungsrichtung. Der Laufzeitunterschied $\Delta t$ wird gemessen und erlaubt die Bestimmung der mittleren Strömungsgeschwindigkeit auf dem vom Schall durchlaufenen Pfad. Durch eine Profilkorrektur kann das Flächenmittel der Strömungsgeschwindigkeit errechnet werden, welches dem Betriebsvolumenstrom proportional ist. Gleichzeitig wird die Gesamtlaufzeit vor und gegen die Strömungsrichtung gemessen, woraus sich dann mit den Geometrieparametern der Installation die Schallgeschwindigkeit des Fluids berechnen lässt. Da Ultraschallwellen auch Festkörper durchdringen, können die Sensoren auf der Außenwand des Rohres befestigt werden. Die Messung funktioniert daher eingriffsfrei und erfordert keinerlei Rohrarbeiten zur Installation der Sensoren und zur Inbetriebnahme des Ultraschallmesssystems.

Bei der Durchflussmessung von Gasen ist es erforderlich, den Betriebsvolumenstrom in einen Standardvolumenstrom oder einen Massestrom umzurechnen, um eine Volumenänderungen durch Temperatur- und Druckeinflüsse herauszurechnen. Dies ist möglich, wenn die Gaszusammensetzung sowie Druck und Temperatur an der Messstelle bekannt sind. Oft ist die Gaszusammensetzung nicht oder nur ungenau bekannt. Dann kann unter Verwendung der Schallgeschwindigkeit und eines empirischen Modells, das an die Bedingungen der Messstelle angepasst ist, der Standardvolumenstrom oder Massestrom auch ohne genaue Kenntnis der Gaszusammensetzung berechnet werden.

Die Parameter werden in Form eines Datensatzes hinterlegt. Ein Datensatz beschreibt eine Gruppe "ähnlicher" Gase, z.B. Natural Gas H. Ein Datensatz wird mit Funktionen, Koeffizienten und/oder Zustandsgrößen beschrieben.

Maschinelles Lernen ist ein Oberbegriff für die "künstliche" Generierung von Wissen aus Erfahrung: Ein künstliches System lernt aus Beispielen und kann diese nach Beendigung der Lernphase verallgemeinern. Dazu bauen Algorithmen beim maschinellen Lernen ein statistisches Modell auf, das auf Trainingsdaten beruht und welches gegen die Testdaten getestet wird. Das heißt, es werden nicht einfach die Beispiele auswendig gelernt, sondern Muster und Gesetzmäßigkeiten in den Lerndaten erkannt. So kann das System auch unbekannte Daten beurteilen.

Die NIST REFPROP-Datenbank bietet die genauesten thermophysikalischen Eigenschaftsmodelle für eine Vielzahl von industriell wichtigen Flüssigkeiten und Flüssigkeitsgemischen, einschließlich anerkannter Standards. Sie hat sich als äußerst nützliches Werkzeug erwiesen, das von der Industrie, der Regierung und der akademischen Welt genutzt wird und thermophysikalische Eigenschaften von reinen Flüssigkeiten und Mischungen über einen weiten Bereich von Flüssigkeitsbedingungen einschließlich flüssiger, gasförmiger und überkritischer Phasen liefert. Sie enthält kritisch bewertete mathematische Modelle mit dem Ziel, die Eigenschaften innerhalb der Unsicherheit der zugrundeliegenden experimentellen Daten, die bei ihrer Entwicklung verwendet wurden, darzustellen. Diese Eigenschaften (wie Dichte, Dampfdruck, Viskosität, Enthalpie usw.) können zur Entwicklung von Lösungen für die Herausforderungen des sich ändernden Klimas sowie zur Bereitstellung genauer thermophysikalischer Daten für Nutzer in der chemischen Industrie, der Erdölindustrie und der Wissenschaft verwendet werden. Im Laufe der Jahre hat sich der Anwendungsbereich der Datenbank von dem ursprünglichen Schwerpunkt Kältemittel zu einer viel breiteren Palette von Chemikalien ausgeweitet; so enthält sie derzeit Normen, die für die Erdgasindustrie (GERG und AGA), Flüssigkeiten, die in Prozessen zur Abscheidung und Sequestrierung von Kohlendioxid eingesetzt werden, Kryogene, die in der Raumfahrt verwendet werden, weit verbreitete Industriechemikalien und Lösungsmittel, Luft, Wasser und Dampf, Bestandteile von Biokraftstoffen und Bestandteile von Ersatzstoffen für komplexe Flüssigkeiten wie Diesel und Düsentreibstoffe.

Die Patentschrift WO2020/112731 offenbart ein Durchflussmesssystem aufweisend einen Durchflussmesser, der mit einer Vielzahl von Sensoren gekoppelt und so konfiguriert ist, dass er das Volumen des durch den Durchflussmesser fließenden Fluids misst. Das System umfasst auch einen Messcomputer, der mit dem Durchflussmesser und den Sensoren verbunden ist. Der Messcomputer ist so konfiguriert, dass er Live-Werte von mehreren Sensoren während eines ersten Zeitraums empfängt, eine Maschine mit künstlicher Intelligenz auf der Grundlage der während des ersten Zeitraums empfangenen Live-Werte trainiert und einen Sensorausfall auf der Grundlage einer Abweichung zwischen einem Live-Wert von dem Sensor und einem vorhergesagten Wert für den Sensor erkennt. Der vorhergesagte Wert basiert auf Live-Werten von anderen Sensoren aus der Vielzahl von Sensoren und der künstlichen Intelligenzmaschine.

In der Veröffentlichung "FLUXUS Natural Gas Engine", 38th International North Sea Flow Measurement Workshop, 26-29 October 2020, G. Hoheisel et al. wird ein Clamp-On-Durchflussmesser offenbart, mittels welchem sich thermodynamische Zustandsgrößen aus den Eingangsmessungen von Druck und Temperatur sowie der direkt gemessenen Schallgeschwindigkeit ableiten und ermöglichen so die Berechnung von Normvolumenstrom oder Massenstrom. Das implementierte Modell des Erdgasmotors vergleicht diese Berechnungen mit den Ergebnissen klassischer Strömungsrechner. Mehr als 2000 Erdgase wurden statistisch ausgewertet. Die Erdgase wurden in Klassen eingeteilt. Mit einer entsprechend identifizierten Fluidklasse kann das im Durchflussmesser eingebettete System die Kompressibilität und das Molekulargewicht einer unbekannten und wechselnden Erdgaszusammensetzung unter Betriebsbedingungen berechnen. Bei zusätzlich eingespeistem Kohlendioxid und Stickstoff lassen sich auch die Brennwerte der Erdgasmischungen berechnen.

In der Veröffentlichungsschrift DE 10 2015 117 468 A1 ist offenbart wie aus der Kombination verschiedener gemessener Summenparameter, wie z.B. Viskosität plus Dichte oder Viskosität plus Schallgeschwindigkeit der Brennwert eines Erdgases bestimmt werden kann. Hierbei wird ein Ansatz der statistischen Analyse der physikalischen Eigenschaften von mehreren tausend Erdgasproben der REFPROP-Datenbank verwendet.

CN 1 12 345 636 A offenbart ein Verfahren zur Berechnung von Erdgaskomponenten auf Basis von Temperatur, Druck und Schallgeschwindigkeit.

WO 95 / 18 958 A1 offenbart ein Verfahren zur Bestimmung des Volumenflusses eines Gases in einem Messrohr.

WO 2023 / 114 382 A1 offenbart Verfahren und Geräte zur Messung von Blasenströmungen von Flüssigkeiten an oder nahe ihrem Übergangspunkt.

US 2003/114992 A1 offenbart eine Methode zur Bestimmung der thermodynamischen Eigenschaften eines Erdgas-Kohlenwasserstoffs, wenn die Schallgeschwindigkeit im Gas bei einer beliebigen Temperatur und einem beliebigen Druck bekannt ist. Die bekannten Parameter sind also Schallgeschwindigkeit, Temperatur, Druck und Konzentration aller verdünnten Bestandteile des Gases. Die Methode verwendet eine Reihe von Referenzgasen und deren berechnete Dichte- und Schallgeschwindigkeitswerte, um die Dichte des betreffenden Gases zu schätzen.

EP 1 063 525 A2 offenbart ein Verfahren zum Messen und/oder Regeln einer in einem Brenngas enthaltenen Wärmemenge, wobei als Eingangsparameter der Heizwert des Brenngases verwendet wird, bei welchem Verfahren

a) das Brenngas oder ein Teilstrom des Brenngases durch einen Volumenzähler oder einen Massenstromzähler geleitet und der Volumenstrom oder der Massenstrom gemessen wird,

b) die Schallgeschwindigkeit des Gases unter ersten Referenzbedingungen bestimmt wird,

c) eine der Messgrößen Dielektrizitätskonstante, Schallgeschwindigkeit unter zweiten Referenzbedingungen, Kohlendioxidgehalt des Brenngases, Stickstoffgehalt des Brenngases oder Dichte unter standardisierten Bedingungen erfasst wird; und

d) aus diesen Parametern wird zusammen mit dem Heizwert des Brenngases die zugeführte Wärmemenge als Messgröße bzw. Regelgröße abgeleitet.

## Darstellung der Erfindung

Es ist Aufgabe der Erfindung die Nachteile des Standes der Technik zu beseitigen sowie ein computerimplementiertes Verfahren zur Ultraschalldurchflussmessung für Gase, insbesondere Erdgase, bereit zu stellen, mittels welchem eine Betriebsvolumenstrommessung in eine Standardvolumenstrom- oder Massestrommessung umgerechnet werden kann, ohne dass dazu die genaue Gaszusammensetzung bzw. Gasbeschaffenheit bekannt ist.

Eine Anpassung von Parametern des computerimplementierten Verfahrens an die Bedingungen der Messstelle soll hierbei automatisch, insbesondere mittels maschinellen Lernens, erfolgen. Die Bedingungen der Messstelle sind der an der Messstelle herrschende Temperatur- und Druck-Bereich sowie die Gasbeschaffenheit.

Die Lösung dieser Aufgabe erfolgt durch die in den Ansprüchen aufgeführten Merkmale.

Die Lösung der Aufgabe erfolgt durch ein computerimplementiertes Verfahren zur Ultraschalldurchflussmessung eines Gases an einer Messstelle mittels eines Ultraschalldurchflussmessgerätes, ohne dass dabei Kenntnisse einer genauen Zusammensetzung des Gases notwendig sind. Das Verfahren umfasst die folgenden Schritte:

a. Erstellen und Abspeichern eines Datensatzes von Testgasen, von Zustandsgrößen $S_{\_test}$ der Testgase und deren Gasbeschaffenheiten in einem Datenspeichergerät,

b. Erstellen eines Eingabedatensatzes mittels einer Eingabevorrichtung, wobei die Eingabedaten folgendes umfassen:

i. Normbedingungen der Messstelle für Temperatur $T_b$ und Druck $P_b$,

ii. Arbeitspunkt an der Messstelle für Temperatur $T_{OP}$ und Druck $P_{OP}$,

iii. Arbeitsbereich der Messstelle mit Temperatur $(T_{min}, T_{max})$ und Druck $(P_{min}, P_{max})$,

iv. Zumindest eine für die Messstelle typische Gasbeschaffenheit eines Referenzgases, wobei ein Referenzgas ein Gas ist, welches am besten abgebildet werden soll,

c. Bestimmung von Zustandsgrößen $S_{ref}$ des Referenzgases mittels einer Recheneinheit, wobei die Zustandsgrößen $S_{ref}$ zumindest folgende sind: eine Kompressibilität $z_{ref(T.P)}$ im eingegebenen Arbeitsbereich, eine Kompressibilität $z_{ref\_b(T\_b,P\_b)}$ im Bereich der eingegebenen Normbedingungen und/oder ein Brennwert $HHV_{ref\_OP}$ und/oder eine Schallgeschwindigkeit $c_{ref\_OP}$ am eingegebenen Arbeitspunkt,

d. Bestimmung einer Teilmenge an Testgasen mittels der Recheneinheit, wobei die Zustandsgrößen $S_{test}$ der Testgase mit denen unter Schritt c) bestimmten Zustandsgrößen $S_{ref}$ verglichen werden, und wobei diejenigen Testgase für die Teilmenge ausgewählt werden, welche sich in einem zuvor definierten Toleranzbereich der Zustandsgrößen $S_{ref}$ befinden und/oder welche nicht im Toleranzbereich der Gasbeschaffenheit der unter b) iv. gemachten Angaben liegen,

e. Berechnung der Kompressibilitäten $z_{OP}$ aller Testgase der Teilmenge am eingegebenen Arbeitspunkt und Berechnung der Schallgeschwindigkeiten $c_{OP}$ aller Testgase der Teilmenge am eingegebenen Arbeitspunkt mittels der Recheneinheit und Vergleich der Kompressibilitäten $z_{OP}$ mit der Kompressibilität $z_{ref\_OP}$, wobei die Differenzen $z_{OP\_diff}$ der Kompressibilitäten $z_{OP}$ zur Kompressibilität $z_{ref\_op}$ als Funktion von $c_{OP}$ approximiert werden,

f. Aussortieren aller Testgase, deren Funktionswerte außerhalb eines Toleranzbereiches von $z_{OP\_diff}$, liegen,

g. Wiederholen der Schritte e. und f. bis ein Residuum kleiner als ein Vorgabewert $R_{min}$ ist, wobei dieser Vorgabewert ein Startwert von 1% sein könnte, oder eine Mindestanzahl an Testgasen der Teilmenge unterschritten ist, so dass eine Auswahl an Testgasen vorliegt und eine Approximation der Kompressibilität $z$ als Funktion der Schallgeschwindigkeit $c$ und unter Normbedingungen $z_b$ erfolgt,

h. Festlegen geschätzter Regressionsparameter für $z_{OP\_diff}$ und Berechnen erweiterter Zustandsgrößen, wie z.B. die Dichte, Brennwert $HHV_v$, Molare Masse $MW$ und weiterer, mittels der in g. bestimmten Kompressibilität $z$

i. Durchführen einer Betriebsvolumenstrommessung mittels des Ultraschalldurchflussmessgerätes und umrechnen der Betriebsvolumenstrommessung in eine Standardvolumenstrom- oder Massenstrommessung mit den ermittelten erweiterten Zustandsgrößen,

Anschließend kann eine Ausgabe mittels einer Ausgabeeinheit eines aus dem gemessenen Betriebsvolumenstrom ermittelten Standardvolumenstroms und/oder eines Massenstroms und/oder der weiteren Zustandsgrößen erfolgen.

Gemäß verschiedener Ausführungsbeispiele wird der Datensatz von Testgasen zusammen mit den Zustandsgrößen $S_{test}$ der Testgase und deren Gasbeschaffenheiten zufällig und/oder aus Messdaten generiert.

Gemäß verschiedener Ausführungsbeispiele werden die Kompressibilität $z_{ref}(T,P)$ im Arbeitsbereich und die Kompressibilität $z_{ref\_b}(T_b,P_b)$ im Bereich der Normbedingungen approximiert, wobei die Approximation bevorzugt gemäß folgenden Funktionen erfolgt (bevorzugt bis N=2):

$$z_{Ref} = \sum_{i,j}^{N} K_{i,j} p^i T^j \qquad (1)$$

$$z_{Ref,b} = \sum_{i,j}^{N} K_{i,j} p_b^i T_b^j \qquad (2)$$

wobei $K_{i,j}$ die Koeffizienten mit i und j von 0 bis zur Ordnung N sind.

Gemäß verschiedener Ausführungsformen werden die Koeffizienten $K_{i,j}$ bevorzugt durch eine multiple Regression 2. Grades nach P und T mit einem Kriterium minimaler Abweichung von einer der Referenzen NIST, REFPROP, AGA und/oder GERG ermittelt. Weitere Referenzen sind denkbar.

Gemäß verschiedener Ausführungsbeispiele umfasst eine vorgegebene Anzahl von Testgasen der Teilmenge eine Mindestanzahl von drei Testgasen und eine Maximalanzahl von einhundert Testgasen.

Gemäß verschiedener Ausführungsformen umfasst der Toleranzbereich der Zustandsgrößen $S_{ref}$ einen Toleranzbereich um den Brennwert des Referenzgases und/oder einen Toleranzbereich um die Schallgeschwindigkeit des Referenzgases.

Gemäß verschiedener Ausführungsformen erfolgt die Berechnung der Kompressibilitäten $z_{OP}$ aller Testgase der Teilmenge am Arbeitspunkt und Berechnung der Schallgeschwindigkeiten $c_{OP}$ aller Testgase der Teilmenge am Arbeitspunkt mittels der Recheneinheit aus einer der Referenzen NIST, REFPROP, AGA und/oder GERG-Referenz.

Gemäß verschiedener Ausführungsformen wird die Differenz $z_{OP\_diff}$ der Kompressibilitäten $z_{OP}$ am Arbeitspunkt zur Kompressibilität $z_{ref\_OP}$ des Referenzgases mit einer Regressionsanalyse als Funktion von $c_{OP}$ approximiert, wobei für die Berechnung der Kompressibilitäten $z_{OP}$ folgende Funktion verwendet wird, bevorzugt bis zur Ordnung N=2:

$$z_{op} = \sum_{i}^{N} M_i c_{op}^i + z_{Ref} \qquad (3)$$

Wobei als Ergebnis i=N+1 geschätzte Regressionsparameter $M_i$ (Koeffizienten) gefunden werden.

Gemäß verschiedener Ausführungsformen wird, wenn die Mindestanzahl der Teilmenge an Testgasen unterschritten ist, der unter Schritt 1a. eingegebene Datensatz um synthetische Gasbeschaffenheiten erweitert und die Verfahrensschritte 1d. -1g werden wiederholt. Die synthetischen Gasbeschaffenheiten werden hierbei zufällig mittels einer mathematischen Simulation generiert (z.B. mittels einer Monte-Carlo-Simulation), wobei sich bei der Simulation der Gasbeschaffenheit an den Eingabedaten aus Schritt b) orientiert wird.

Wenn die Maximalanzahl der Teilmenge an Testgasen überschritten ist, dann werden gemäß verschiedener Ausführungsformen, die Verfahrensschritte 1d. -1g. wiederholt, wobei der definierte Toleranzbereich T der Zustandsgrößen $S_{ref}$ und/oder der Toleranzbereich des Brennwertes und/oder der Toleranzbereich der Schallgeschwindigkeit reduziert worden ist/sind.

Wenn die Maximalanzahl der Teilmenge an Testgasen überschritten ist, dann werden gemäß verschiedener Ausführungsformen, die Verfahrensschritte 1d. -1g. wiederholt, wobei das Residuum reduziert worden ist.

Gemäß verschiedener Ausführungsformen wird die Kompressibilität z als Funktion der Schallgeschwindigkeit und die Kompressibilität $z_b$ als Funktion der Schallgeschwindigkeit unter Normbedingungen nach Verfahrensschritt g. mittels folgender Gleichungen approximiert:

$$z = \sum_i^N M_i \left( \delta \cdot c \right)^i + z_{Ref} \qquad (4)$$

$$z_b = \sum_i^N M_i \left( \delta_b \cdot c \right)^i + z_{Ref,b} \qquad (5)$$

wobei Faktor $\delta = d(t,p)$ in Gleichung 4 ein Verhältnis der Schallgeschwindigkeit $c_{ref}$ des Referenzgases unter variierenden Prozessbedingungen zur Schallgeschwindigkeit $c_{ref\_OP}$ des Referenzgases am Arbeitspunkt berücksichtigt und wobei Faktor $\delta_b = d(t_b,p_b)$ in Gleichung 5 ein Verhältnis der Schallgeschwindigkeit $c_{ref\_b}$ des Referenzgases unter Normbedingungen zur Schallgeschwindigkeit $c_{ref\_OP}$ des Referenzgases am Arbeitspunkt berücksichtigt.

Gemäß verschiedener Ausführungsformen wird ein Adiabatenkoeffizient g(p,T) des Referenzgases approximiert, mittels folgender Gleichung:

$$\gamma = \sum_{i,j}^N L_{i,j} p^i T^j \qquad (6)$$

wobei Gleichung (6) bevorzugt bis zur Ordnung N=2 approximiert wird, wobei die Approximation durch multiple Regression 2. Grades nach P und T mit dem Kriterium minimaler Abweichung von einer der Referenzen NIST, REFPROP, AGA oder GERG Referenz erfolgt. Mit dem Adiabatenkoeffizienten $\gamma$ (in Abhängigkeit von p und T) wird die molare Masse $\Gamma$ nach folgender Gleichung (7) berechnet:

$$\Gamma = RT \frac{\gamma}{c^2} \qquad (7)$$

wobei R eine universelle Gaskonstante ist.

Eine molare Masse von jedem Testgas wird mittels folgender Gleichung ermittelt:

$$MW = \Gamma + f(c,p) \qquad (8)$$

wobei die Funktion f(c,p) mit einem Ansatz nach Gleichung 9 approximiert wird:

$$f(c,p) = \sum_i^N O_i p^i \cdot \left( \Gamma - M_{Ref} \right) \cdot \frac{2p_{op} - p}{p} \qquad (9)$$

bevorzugt bis zur Ordnung N=2, wobei $M_{Ref}$ eine Molmasse eines Hauptbestandteils des Referenzgases korrigiert mit der gemessenen Schallgeschwindigkeit c ist.

Gemäß verschiedener Ausführungsformen wird eine Betriebsdichte r und/oder eine Dichte rb bei Normbedin-

gungen berechnet mittels

$$\rho = \frac{p \cdot MW}{z \cdot RT} \quad\quad (10)$$

$$\rho_b = \frac{p_b \cdot MW}{z_b RT_b} \quad\quad (11)$$

Für jedes Testgas aus der ermittelten Teilmenge der Testgase wird ein oberer Brennwert $HHV_m$ (higher heating value), eine Molmasse MW und eine Schallgeschwindigkeit $c_{op}$ am Arbeitspunkt aus einer der Referenzen NIST, REFPROP, AGA oder GERG-Referenz berechnet werden, wobei Werte des $HHV_m$ als Funktion von c approximiert werden.

Die Approximation von $HHV_m$ erfolgt bevorzugt mittels folgender Funktion:

$$HHV_m = \left( \sum_i^N Q_i c_{op}^i \right) \cdot \left( 1 - \%CO_2 \cdot \frac{MW_{CO_2}}{MW} - \%N_2 \cdot \frac{MW_{N_2}}{MW} \right) \quad (12)$$

wobei $CO_2$ und $N_2$ Anteile des jeweiligen Testgases eingesetzt werden und wobei $MW_{CO_2}$ und $MW_{N_2}$ die molaren Massen von $CO_2$ und $N_2$ sind. Mit der Dichte bei Normbedingungen $\rho_b$ können die Brennwerte $HHV_m$ bezogen auf die Masse und die Brennwerte $HHV_v$ bezogen auf das Volumen ineinander mittels folgender Gleichung (13) konvertiert werden.

$$HHV_v \quad \text{in } [\text{MJ/m}^3] = \rho_b \cdot HHV_m \quad \text{in } [\text{MJ/kg}] \quad\quad (13)$$

Gemäß verschiedener Ausführungsformen werden die Verfahrensschritte 1d.-1g. erneut durchgeführt, wenn die Eingabedaten aus Verfahrensschritt 1a. neu eingegeben werden.

Die Aufgabe wird auch gelöst mittels einer Vorrichtung zur Durchflussmessung von Gasen mit Mitteln zur Durchführung des Verfahrens umfassend ein Datenspeichergerät, eine Eingabevorrichtung, eine Empfangseinheit, ein Durchflussmessgerät und/oder eine Recheneinheit, wobei die Mittel so angepasst sind, dass sie die Schritte des Verfahrens nach einem der Ansprüche 1 - 24 ausführen.

Die Aufgabe wird auch gelöst mittels eines Computerprogramms mit Befehlen, die, wenn das Computerprogramm von einer Vorrichtung zur Durchflussmessung von Gasen nach Anspruch 26 ausgeführt wird, die Vorrichtung veranlassen, das Verfahren nach einem der Ansprüche 1-24 durchzuführen.

Die Aufgabe wird auch gelöst mittels eines computerlesbaren Medium, welches Anweisungen enthält, die, wenn sie von einer Vorrichtung zur Durchflussmessung von Gasen nach Anspruch 26 ausgeführt werden, die Vorrichtung veranlassen, das Verfahren nach einem der Ansprüche 1 bis 24 auszuführen.

**Ausführung der Erfindung**

Die Erfindung wird anhand eines Ausführungsbeispiels näher erläutert. Hierzu zeigt

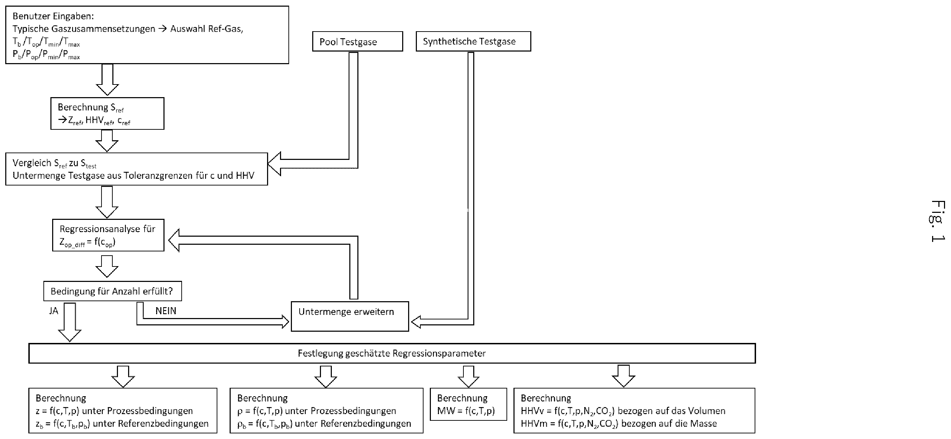

Figur 1    ein Ablaufdiagram des erfindungsgemäßen Verfahrens

In der Beschreibung wird auf die beigefügten Zeichnungen Bezug genommen, in denen zur Veranschaulichung spezifische Ausführungsformen gezeigt sind, in denen die erfindungsgemäße Anordnung ausgeübt werden kann. In dieser Hinsicht wird eine Richtungsterminologie wie etwa "oben", "unten" usw. mit Bezug auf die Orientierung der beschriebenen Zeichnungen verwendet. Die Richtungsterminologie dient der Veranschaulichung und ist auf keinerlei Weise einschränkend.

Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

Figur 1 zeigt ein Ablaufdiagramm des hier vorgestellten erfindungsgemäßen computerimplementierten Verfahrens zur Ultraschalldurchflussmessung eines Gases an einer Messstelle mittels eines Ultraschalldurchflussmessgerätes. Das Verfahren umfasst die Schritte:

a. Erstellen und Abspeichern eines Datensatzes von Testgasen (in Figur 1 "Pool Testgase"), von Zustandsgrößen $S_{test}$ der Testgase und deren Gasbeschaffenheiten in einem Datenspeichergerät, beispielsweise auf einem Speicher eines PCs, eines Messgerätes oder eines Servers. Der Pool an Testgasen kann beispielsweise 2000 Testgase umfassen. Ein Testgas wird darin durch seine Gasbeschaffenheit beschrieben. Eine Teilmenge davon dient der Berechnung eines applikationsspezifischen Datensatzes eines thermodynamischen Gasmodells. Der Testgas-Pool kann zufällig generiert werden oder aus echten Messdaten bestehen.

b. Erstellen eines Eingabedatensatzes mittels einer Eingabevorrichtung, die Eingabedaten von einem Anwender bevorzugt manuell eingegeben werden. Die Eingabedaten umfassen folgende Daten:

i. Normbedingungen der Messstelle für Temperatur $T_b$ und Druck $P_b$,
ii. Arbeitspunkt an der Messstelle für Temperatur $T_{OP}$ und Druck $P_{OP}$,
iii. Arbeitsbereich der Messstelle mit Temperatur ($T_{min}$, $T_{max}$) und Druck ($P_{min}$, $P_{max}$),
iv. Zumindest eine für die Messstelle typische Gasbeschaffenheit eines Referenzgases (in Figur 1 "Auswahl Ref-Gas"), wobei ein Referenzgas ein Gas ist, welches am besten abgebildet werden soll,

c. Bestimmung von Zustandsgrößen $S_{ref}$ des Referenzgases mittels einer Recheneinheit, beispielsweise einem Computer, wobei die Zustandsgrößen $S_{ref}$ zumindest folgende aufweisen: eine Kompressibilität $z_{ref(T.P)}$ im eingegebenen Arbeitsbereich, eine Kompressibilität $z_{ref\_b(T\_b,P\_b)}$ im Bereich der eingegebenen Normbedingungen und/oder ein Brennwert $HHV_{ref\_OP}$ und/oder eine Schallgeschwindigkeit $c_{ref\_OP}$ am eingegebenen Arbeitspunkt,

d. Bestimmung und Auswahl einer Teilmenge an Testgasen aus dem Testgaspool, mittels der Recheneinheit, wobei die Zustandsgrößen $S_{test}$ der Testgase mit denen unter Schritt c) bestimmten Zustandsgrößen $S_{ref}$ verglichen werden, und wobei diejenigen Testgase für die Teilmenge ausgewählt werden, welche sich in einem zuvor definierten Toleranzbereich der Zustandsgrößen $S_{ref}$ befinden und/oder welche nicht im Toleranzbereich der Gasbeschaffenheit der unter b) iv. gemachten Angaben liegen. Die Auswahl erfolgt so, dass das daraus entwickelte Modell ausreichend gut an die spezifische Messstelle angepasst ist, aber trotzdem breitbandig genug ist, die eingegebenen Anwender-Randbedingungen noch zu erweitern, um numerisch stabil zu bleiben. Weiterhin soll die Anzahl der Testgase innerhalb eines vorgegebenen Bereiches liegen, z.B. mindestens 3 und maximal 100.

e. Berechnung der Kompressibilitäten $z_{OP}$ aller Testgase der Teilmenge am eingegebenen Arbeitspunkt und Berechnung der Schallgeschwindigkeiten $c_{OP}$ aller Testgase der Teilmenge am eingegebenen Arbeitspunkt mittels der Recheneinheit und Vergleich der Kompressibilitäten $z_{OP}$ mit der Kompressibilität $z_{ref\_OP}$, wobei die Differenzen $z_{OP\_diff}$ der Kompressibilitäten $z_{OP}$ zur Kompressibilität $z_{ref\_op}$ als Funktion von $c_{OP}$ approximiert werden. Es erfolgt hier eine Regressionsanalyse für $z_{OP\_diff} = f(c_{OP})$.

f. Aussortieren aller Testgase, deren Funktionswerte außerhalb eines Toleranzbereiches von $z_{OP\_diff}$, liegen.

g. Wiederholen der Schritte e. und f. bis ein Residuum kleiner als ein Vorgabewert $R_{min}$ ist oder eine Mindestanzahl an Testgasen der Teilmenge unterschritten ist, so dass eine Auswahl an Testgasen vorliegt und eine Approximation der Kompressibilität z als Funktion der Schallgeschwindigkeit c und unter Normbedingungen $z_b$ erfolgt,

h. Festlegen geschätzter Regressionsparameter für $z_{OP\_diff}$ und Berechnen erweiterter Zustandsgrößen, wie z.B. die Dichte, Brennwert $HHV_v$, Molare Masse MW und weiterer, mittels der in g. bestimmten Kompressibilität z Durchführen einer Betriebsvolumenstrommessung mittels des Ultraschalldurchflussmessgerätes und umrechnen der Betriebsvolumenstrommessung in eine Standardvolumenstrom- oder Massenstrommessung mit den ermittelten erweiterten Zustandsgrößen.

Anschließend kann eine Ausgabe mittels einer Ausgabeeinheit eines aus dem bestimmten Betriebsvolumenstrom ermittelten Standardvolumenstroms und/oder eines Massenstroms und/oder der erweiterten Zustandsgrößen erfolgen.

**Patentansprüche**

1. Computerimplementiertes Verfahren zur Ultraschalldurchflussmessung eines Gases an einer Messstelle mittels eines Ultraschalldurchflussmessgerätes, ohne Kenntnis einer Zusammensetzung des Gases/Fluids, umfassend die Schritte:

a. Erstellen und Abspeichern eines Datensatzes von Testgasen, von Zustandsgrößen $S_{test}$ der Testgase und deren Gasbeschaffenheiten in einem Datenspeichergerät,

b. Erstellen eines Eingabedatensatzes mittels einer Eingabevorrichtung, wobei die Eingabedaten folgendes umfassen:

  i. Normbedingungen der Messstelle für Temperatur $T_b$ und Druck $P_b$,
  ii. Arbeitspunkt an der Messstelle für Temperatur $T_{OP}$ und Druck $P_{OP}$
  iii. Arbeitsbereich der Messstelle mit Temperatur ($T_{min}$, $T_{max}$) und Druck ($P_{min}$, $P_{max}$),
  iv. Zumindest eine für die Messstelle typische Gasbeschaffenheit und Auswahl einer konkreten Gasbeschaffenheit eines Referenzgases

c. Bestimmung von Zustandsgrößen $S_{ref}$ des Referenzgases mittels einer Recheneinheit, wobei die Zustandsgrößen $S_{ref}$ zumindest folgende sind: eine Kompressibilität $z_{ref(T.P)}$ im Arbeitsbereich, eine Kompressibilität $z_{ref\_b(T\_b,P\_b)}$ im Bereich der Normbedingungen und/oder ein Brennwert $HHV_{ref\_OP}$ und/oder eine Schallgeschwindigkeit $C_{ref\_OP}$ am Arbeitspunkt,

d. Bestimmung einer Teilmenge an Testgasen mittels der Recheneinheit, wobei die Zustandsgrößen $S_{test}$ der Testgase mit denen unter Schritt c) bestimmten Zustandsgrößen $S_{ref}$ verglichen werden, und wobei diejenigen Testgase für die Teilmenge ausgewählt werden, welche sich in einem zuvor definierten Toleranzbereich der Zustandsgrößen $S_{ref}$ befinden und/oder welche nicht im Toleranzbereich der Gasbeschaffenheit der unter b) iv. gemachten Angaben liegen,

e. Berechnung der Kompressibilitäten $z_{OP}$ aller Testgase der Teilmenge am Arbeitspunkt und Berechnung der Schallgeschwindigkeiten $c_{OP}$ aller Testgase der Teilmenge am Arbeitspunkt mittels der Recheneinheit und Vergleich der Kompressibilitäten $z_{OP}$ mit der Kompressibilität $z_{ref\_OP}$, wobei die Differenzen $z_{OP\_diff}$ der Kompressibilitäten $z_{OP}$ zur Kompressibilität $z_{ref\_op}$ als Funktion von $c_{OP}$ approximiert werden,

f. Aussortieren aller Funktionswerte, welche außerhalb eines Toleranzbereiches der approximierten Funktion liegen,

g. Wiederholen der Schritte e. und f. bis ein Residuum kleiner als ein Vorgabewert $R_{min}$ ist oder eine Mindestanzahl an Testgasen unterschritten ist, so dass eine Auswahl an Testgasen vorliegt und eine Approximation der Kompressibilität z als Funktion der Schallgeschwindigkeit c und unter Normbedingungen $z_b$ erfolgt,

h. Festlegen geschätzter Regressionsparameter für $z_{OP\_diff}$ und Berechnen erweiterter Zustandsgrößen mittels der in g. bestimmten Kompressibilität z,

i. Durchführen einer Betriebsvolumenstrommessung mittels des Ultraschalldurchflussmessgerätes und umrechnen der Betriebsvolumenstrommessung in eine Standardvolumenstrom- oder Massenstrommessung mit den ermittelten erweiterten Zustandsgrößen.

2. Computerimplementiertes Verfahren gemäß Anspruch 1, **dadurch gekennzeichnet, dass** die Testgase zufällig generiert werden und/oder aus Messdaten generiert werden.

3. Computerimplementiertes Verfahren gemäß einem der vorhergehenden Ansprüche, **dadurch gekennzeichnet, dass** die erweiterten Zustandsgrößen eine Dichte, ein Brennwert $HHV_v$ und/oder eine Molare Masse MW umfassen.

4. Computerimplementiertes Verfahren gemäß Anspruch 1 oder 2, **dadurch gekennzeichnet, dass** die Kompressibilität $z_{ref(T,P)}$ im Arbeitsbereich und die Kompressibilität $z_{ref\_b(T\_b,P\_b)}$ im Bereich der Normbedingungen approximiert werden.

5. Computerimplementiertes Verfahren gemäß Anspruch 4, **dadurch gekennzeichnet, dass** die Approximation gemäß folgenden Funktionen erfolgt (bevorzugt bis N=2):

$$z_{Ref} = \sum_{i,j}^{N} K_{i,j} p^i T^j \qquad (1)$$

$$z_{Ref,b} = \sum_{i,j}^{N} K_{i,j} p_b^i T_b^j \qquad (2)$$

wobei $K_{i,j}$ die Koeffizienten mit i und j von 0 bis zur Ordnung N sind.

6. Computerimplementiertes Verfahren gemäß Anspruch 5, **dadurch gekennzeichnet, dass** die Koeffizienten durch eine multiple Regression 2. Grades nach P und T mit einem Kriterium minimaler Abweichung von der NEST/REFPROP/AGA/GERG-Referenz ermittelt werden.

7. Computerimplementiertes Verfahren gemäß einem der vorhergehenden Ansprüche, **dadurch gekennzeichnet, dass** die Anzahl der Testgase innerhalb eines vorgegebenen Bereiches liegen.

8. Computerimplementiertes Verfahren gemäß Anspruch 7, **dadurch gekennzeichnet, dass** der vorgegebene Bereich eine Mindestanzahl von 3 und eine Maximalanzahl von 100 Testgasen umfasst.

9. Computerimplementiertes Verfahren gemäß einem der vorhergehenden Ansprüche, **dadurch gekennzeichnet, dass**
   der Toleranzbereich T der Zustandsgrößen $S_{ref}$ einen Toleranzbereich um den Brennwertes des Referenzgases und/oder einen Toleranzbereich um die Schallgeschwindigkeit des Referenzgases umfasst.

10. Computerimplementiertes Verfahren gemäß einem der vorhergehenden Ansprüche, **dadurch gekennzeichnet, dass**
    die Berechnung der Kompressibilitäten $z_{OP}$ aller Testgase der Teilmenge am Arbeitspunkt und Berechnung der Schallgeschwindigkeiten $c_{OP}$ aller Testgase der Teilmenge am Arbeitspunkt mittels der Recheneinheit aus der NIST/REFPROP/AGA/GERG-Referenz erfolgt.

11. Computerimplementiertes Verfahren gemäß einem der vorhergehenden Ansprüche, **dadurch gekennzeichnet, dass**
    die Differenz $z_{OP\_diff}$ der Kompressibilitäten $z_{OP}$ am Arbeitspunkt zur Kompressibilität $z_{ref\_OP}$ des Referenzgases mit einer Regressionsanalyse als Funktion von $c_{OP}$ approximiert werden, wobei folgende Funktion verwendet wird, bevorzugt bis zur Ordnung N=2:

$$z_{op} = \sum_{i}^{N} M_i c_{op}^i + z_{Ref} \qquad (3)$$

12. Computerimplementiertes Verfahren gemäß einem der vorhergehenden Ansprüche, **dadurch gekennzeichnet, dass**
    wenn eine Mindestanzahl der Teilmenge an Testgasen unterschritten ist, wird der unter Schritt 1a. eingegebene Datensatz um synthetische Gasbeschaffenheiten erweitert und die Verfahrensschritte 1d. - 1g. wiederholt.

13. Computerimplementiertes Verfahren gemäß einem der vorhergehenden Ansprüche, **dadurch gekennzeichnet, dass**
    wenn eine Maximalanzahl der Teilmenge an Testgasen überschritten ist, dann werden die Verfahrensschritte 1d. - 1g. wiederholt, wobei der definierte Toleranzbereich T der Zustandsgrößen $S_{ref}$ und/oder der Toleranzbereich des Brennwertes und/oder der Toleranzbereich der Schallgeschwindigkeit reduziert ist/sind.

14. Computerimplementiertes Verfahren gemäß Anspruch 1, **dadurch gekennzeichnet, dass**
    wenn eine Maximalanzahl der Teilmenge an Testgasen überschritten ist, dann werden die Verfahrensschritte 1d. - 1g. wiederholt, wobei das Residuum reduziert ist.

15. Computerimplementiertes Verfahren gemäß einem der vorhergehenden Ansprüche, **dadurch gekennzeichnet,**

**dass**

die Kompressibilität z als Funktion der Schallgeschwindigkeit und unter Normbedingungen nach Verfahrensschritt g. mittels folgender Gleichungen approximiert wird:

$$z = \sum_{i}^{N} M_i \left(\delta \cdot c\right)^i + z_{Ref} \qquad (4)$$

$$z_b = \sum_{i}^{N} M_i \left(\delta_b \cdot c\right)^i + z_{Ref,b} \qquad (5)$$

wobei Faktor $\delta = d(t,p)$ in Gleichung 4 ein Verhältnis der Schallgeschwindigkeit $c_{ref}$ des Referenzgases unter variierenden Prozessbedingungen zur Schallgeschwindigkeit $c_{ref\_OP}$ des Referenzgases am Arbeitspunkt berücksichtigt und wobei Faktor $\delta_b = d(t_b,p_b)$ in Gleichung 5 ein Verhältnis der Schallgeschwindigkeit $c_{ref\_b}$ des Referenzgases unter Normbedingungen zur Schallgeschwindigkeit $c_{ref\_OP}$ des Referenzgases am Arbeitspunkt berücksichtigt.

16. Computerimplementiertes Verfahren gemäß einem der vorhergehenden Ansprüche, **dadurch gekennzeichnet, dass**
ein Adiabatenkoeffizient g(p,T) des Referenzgases approximiert wird mittels folgender Gleichung:

$$\gamma = \sum_{i,j}^{N} L_{i,j} p^i T^j \qquad (6)$$

17. Computerimplementiertes Verfahren gemäß Anspruch 16, **dadurch gekennzeichnet, dass**
Gleichung (6) bevorzugt bis zur Ordnung N=2 approximiert wird, wobei die Approximation durch multiple Regression 2. Grades nach P und T mit dem Kriterium minimaler Abweichung von der NIST/REFPROP/AGA/GERG Referenz erfolgt.

18. Computerimplementiertes Verfahren gemäß Anspruch 16 oder 17, **dadurch gekennzeichnet, dass**

mit dem Adiabatenkoeffizienten $\gamma$ (p,T) eine molare Masse $\Gamma$ nach folgender Gleichung (7) berechnet wird:

$$\Gamma = RT \frac{\gamma}{c^2} \qquad (7)$$

wobei R eine universelle Gaskonstante ist.

19. Computerimplementiertes Verfahren gemäß Anspruch 18, **dadurch gekennzeichnet, dass**
eine molare Masse MW von jedem Testgas mittels folgender Gleichung ermittelt wird:

$$MW = \Gamma + f(c,p) \qquad (8)$$

20. Computerimplementiertes Verfahren gemäß Anspruch 19, **dadurch gekennzeichnet, dass**

f(c,p) mit einem Ansatz nach Gleichung 9 approximiert wird:

$$f(c,p) = \sum_{i}^{N} O_i p^i \cdot (\Gamma - M_{Ref}) \cdot \frac{2p_{op} - p}{p} \qquad (9)$$

bevorzugt bis zur Ordnung N=2, wobei $M_{Ref}$ eine Molmasse eines Hauptbestandteils des Referenzgases korrigiert mit der gemessenen Schallgeschwindigkeit c ist.

21. Computerimplementiertes Verfahren gemäß Anspruch 19, **dadurch gekennzeichnet, dass** eine Betriebsdichte p und/oder eine Dichte $p_b$ bei Normbedingungen berechnet werden mittels

$$\rho = \frac{p \cdot MW}{z \cdot RT} \qquad (10)$$

$$\rho_b = \frac{p_b \cdot MW}{z_b RT_b} \qquad (11)$$

22. Computerimplementiertes Verfahren gemäß einem der vorhergehenden Ansprüche, **dadurch gekennzeichnet, dass** für jedes Testgas aus der ermittelten Teilmenge aller Testgase ein oberer Brennwert $HHV_m$ (higher heating value), eine Molmasse MW und eine Schallgeschwindigkeit $c_{op}$ am Arbeitspunkt aus der NIST/REFPROP/AGA/GERG-Referenz berechnet werden, wobei Werte des $HHV_m$ als Funktion von c approximiert werden.

23. Computerimplementiertes Verfahren gemäß Anspruch 22, **dadurch gekennzeichnet, dass**

die Approximation von $HHV_m$ mittels folgender Funktion erfolgt:

$$HHV_m = \left( \sum_{i}^{N} Q_i c_{op}^i \right) \cdot \left( 1 - \%CO_2 \cdot \frac{MW_{CO_2}}{MW} - \%N_2 \cdot \frac{MW_{N_2}}{MW} \right) \quad (12)$$

wobei $CO_2$ und $N_2$ Anteile des jeweiligen Testgases eingesetzt werden und wobei $MW_{CO_2}$ und $MW_{N2}$ die molaren Massen von $CO_2$ und $N_2$ sind.

24. Computerimplementiertes Verfahren gemäß Anspruch 23, **dadurch gekennzeichnet, dass** $HHV_V$ berechnet wird mittels

$$HHV_v \quad \text{in } [\text{MJ/m}^3] = \rho_b \cdot HHV_m \quad \text{in } [\text{MJ/kg}] \qquad (13)$$

25. Computerimplementiertes Verfahren gemäß einem der vorhergehenden Ansprüche, **dadurch gekennzeichnet, dass** die Verfahrensschritte 1d.-1g. erneut durchgeführt werden, wenn die Eingabedaten aus Verfahrensschritt 1a. neu eingegeben werden.

26. Vorrichtung zur Durchflussmessung von Gasen mit Mitteln zur Durchführung des Verfahrens umfassend ein Datenspeichergerät, eine Eingabevorrichtung, eine Empfangseinheit, ein Durchflussmessgerät und/oder eine Recheneinheit, wobei die Mittel so angepasst sind, dass sie die Schritte des Verfahrens nach einem der Ansprüche 1 - 24 ausführen.

27. Computerprogramm mit Befehlen, die, wenn das Programm von einer Vorrichtung zur Durchflussmessung von Gasen nach Anspruch 26 ausgeführt wird, die Vorrichtung veranlassen, das Verfahren nach einem der Ansprüche 1-24 durchzuführen.

28. Computerlesbares Medium, das Anweisungen enthält, die, wenn sie von einer Vorrichtung zur Durchflussmessung von Gasen nach Anspruch 26 ausgeführt werden, die Vorrichtung veranlassen, das Verfahren nach einem der Ansprüche 1 bis 24 auszuführen.

**Claims**

1. Computer-implemented method for ultrasonic flow measurement of a gas at a measuring point by means of an ultrasonic flow meter, without knowledge of a composition of the gas/fluid, comprising the steps:

    a. creating and storing a data set of test gases, of state variables $S_{test}$ of the test gases and their gas qualities in a data storage device,
    b. creating an input data set using an input device, the input data comprising the following:

        i. standard conditions of the measuring point for temperature $T_b$ and pressure $P_b$,
        ii. operating point at the measuring point for temperature $T_{OP}$ and pressure $P_{OP}$
        iii. working range of the measuring point with temperature $(T_{min}, T_{max})$ and pressure $(P_{min}, P_{max})$,
        iv. at least one gas quality typical for the measuring point and selection of a specific gas quality of a reference gas,

    c. determining state variables $S_{ref}$ of the reference gas by means of a computing unit, wherein the state variables $S_{ref}$ are at least the following: a compressibility $z_{ref(T,P)}$ in the working range, a compressibility $Z_{ref\_b(T\_b.P\_b)}$ in the range of the standard conditions and/or a heating value $HHV_{ref\_OP}$ and/or a sound velocity $C_{ref\_OP}$ at the operating point,
    d. determining a partial quantity of test gases by means of the computing unit, wherein the state variables $S_{test}$ of the test gases are compared with the state variables $S_{ref}$ determined in step c), and wherein those test gases are selected for the partial quantity which are within a previously defined tolerance range of the state variables $S_{ref}$ and/or which are not within the tolerance range of the gas quality of the specifications made under b) iv.;
    e. calculating the compressibilities $Z_{OP}$ of all test gases of the partial quantity at the operating point and calculating the sound velocities $c_{OP}$ of all test gases of the partial quantity at the operating point by means of the computing unit and comparing the compressibilities $Z_{OP}$ with the compressibility $Z_{ref\_OP}$, wherein the differences $z_{OP\_diff}$ of the compressibilities $Z_{OP}$ to the compressibility $Z_{ref\_OP}$ are approximated as a function of $C_{OP}$,
    f. sorting out all function values that lie outside a tolerance range of the approximated function,
    g. repeating steps e. and f. until a residual is less than a specified value $R_{min}$ or a minimum number of test gases is not reached, so that a selection of test gases is available and an approximation of the compressibility z is carried out as a function of the sound velocity c and under standard conditions $Z_b$,
    h. setting estimated regression parameters for $z_{OP\_diff}$ and calculating extended state variables using the compressibility z determined in g.,
    i. Carrying out an operating volume flow measurement using the ultrasonic flow meter and converting the operating volume flow measurement into a standard volume flow measurement or mass flow measurement with the determined extended state variables.

2. Computer-implemented method according to claim 1, **characterised in that** the test gases are randomly generated and/or are generated from measurement data.

3. Computer-implemented method according to one of the preceding claims, **characterised in that** the extended state variables comprise a density, a heating value $HHV_v$ and/or a molar mass MW.

4. Computer-implemented method according to claim 1 or 2, **characterised in that** the compressibility $z_{ref(T,P)}$ in the working range and the compressibility $Z_{ref\_b(T\_b,P\_b)}$ in the range of the standard conditions are approximated.

5. Computer-implemented method according to claim 4, **characterised in that** the approximation is carried out according to the following functions (preferably up to N=2):

$$z_{Ref} = \sum_{i,j}^{N} K_{i,j} p^i T^j \qquad (1)$$

$$z_{Ref,b} = \sum_{i,j}^{N} K_{i,j} p_b^i T_b^j \qquad (2)$$

wherein $K_{i,j}$ are the coefficients with i and j from 0 to the order N.

6.  Computer-implemented method according to claim 5, **characterised in that** the coefficients are determined through a 2nd degree multiple regression according to P and T with a criterion of minimal deviation from the NEST/REFPROP/AGA/GERG reference.

7.  Computer-implemented method according to one of the preceding claims, **characterised in that** the number of test gases is within a specified range.

8.  Computer-implemented method according to claim 7, **characterised in that** the specified range comprises a minimum number of 3 and a maximum number of 100 test gases.

9.  Computer-implemented method according to one of the preceding claims, **characterised in that** the tolerance range T of the state variables $S_{ref}$ comprises a tolerance range around the heating value of the reference gas and/or a tolerance range around the sound velocity of the reference gas.

10. Computer-implemented method according to one of the preceding claims, **characterised in that** the calculation of the compressibilities $Z_{OP}$ of all test gases of the partial quantity at the operating point and calculation of the sound velocities $C_{OP}$ of all test gases of the partial quantity at the operating point is carried out using the computing unit from the NIST/REFPROP/AGA/GERG reference.

11. Computer-implemented method according to one of the preceding claims, **characterised in that** the difference $z_{OP\_diff}$ of the compressibilities $Z_{OP}$ at the operating point to the compressibility $Z_{ref\_OP}$ of the reference gas are approximated as a function of $C_{OP}$ with a regression analysis, wherein the following function is used, preferably up to the order N =2:

$$z_{op} = \sum_{i}^{N} M_i c_{op}^i + z_{Ref} \qquad (3)$$

12. Computer-implemented method according to one of the preceding claims, **characterised in that** if a minimum number of the partial quantity of test gases is not reached, the data set entered under step 1a. is extended to include synthetic gas qualities and the method steps 1d.-1g. are repeated.

13. Computer-implemented method according to one of the preceding claims, **characterised in that** if a maximum number of the partial quantity of test gases is exceeded, then the method steps 1d.-1g. are repeated, wherein the defined tolerance range T of the state variables $S_{ref}$ and/or the tolerance range of the heating value and/or the tolerance range of the sound velocity is/are reduced.

14. Computer-implemented method according to claim 1, **characterised in that** if a maximum number of the partial quantity of test gases is exceeded, then the method steps 1d.-1g. are repeated, wherein the residual is reduced.

15. Computer-implemented method according to one of the preceding claims, **characterised in that** the compressibility z is approximated as a function of the sound velocity and under standard conditions according to method step g. by means of the following equations:

$$z = \sum_{i}^{N} M_i \left(\delta \cdot c\right)^i + z_{Ref} \tag{4}$$

$$z_b = \sum_{i}^{N} M_i \left(\delta_b \cdot c\right)^i + z_{Ref,b} \tag{5}$$

wherein factor $\delta = d(t,p)$ in equation 4 takes into account a ratio of the sound velocity $c_{ref}$ of the reference gas under varying process conditions to the sound velocity $C_{ref\_OP}$ of the reference gas at the operating point, and wherein factor $\delta b = d(t_b,p_b)$ in equation 5 takes into account a ratio of the sound velocity $C_{ref\_b}$ of the reference gas under standard conditions to the sound velocity $C_{ref\_OP}$ of the reference gas at the operating point.

16. Computer-implemented method according to one of the preceding claims, **characterised in that** an adiabatic coefficient $g(p,T)$ of the reference gas is approximated by means of the following equation:

$$\gamma = \sum_{i,j}^{N} L_{i,j} p^i T^j \tag{6}$$

17. Computer-implemented method according to claim 16, **characterised in that** equation (6) is preferably approximated up to the order N=2, wherein the approximation is carried out through multiple regression of the 2nd degree according to P and T with the criterion of minimal deviation from the NIST/REFPROP/AGA/GERG reference.

18. Computer-implemented method according to claim 16 or 17, **characterised in that**, with the adiabatic coefficient $\gamma$ $(p,T)$, a molar mass $\Gamma$ is calculated according to the following equation (7):

$$\Gamma = RT \frac{\gamma}{c^2} \tag{7}$$

wherein R is a universal gas constant.

19. Computer-implemented method according to claim 18, **characterised in that** a molar mass MW of each test gas is determined by means of the following equation:

$$MW = \Gamma + f(c,p) \tag{8}$$

20. Computer-implemented method according to claim 19, **characterised in that** $f(c,p)$ is approximated using an estimate according to
Equation 9:

$$f(c,p) = \sum_{i}^{N} O_i p^i \cdot \left(\Gamma - M_{Ref}\right) \cdot \frac{2p_{op} - p}{p} \tag{9}$$

preferably up to the order N=2, wherein $M_{Ref}$ is a molar mass of a main constituent of the reference gas corrected with the measured sound velocity c.

21. Computer-implemented method according to claim 19, **characterised in that** an operating density p and/or a density Pb under standard conditions are calculated by means of

$$\rho = \frac{p \cdot MW}{z \cdot RT} \qquad (10)$$

$$\rho_b = \frac{p_b \cdot MW}{z_b RT_b} \qquad (11)$$

**22.** Computer-implemented method according to one of the preceding claims, **characterised in that** for each test gas from the determined partial quantity of all test gases, a higher heating value HHVm, a molar mass MW and a sound velocity $C_{OP}$ at the operating point are calculated from the NIST/REFPROP/AGA/GERG reference, wherein values of the HHVm are approximated as a function of c.

**23.** Computer-implemented method according to claim 22, **characterised in that** the approximation of HHVm is carried out by means of the following function:

$$HHV_m = \left( \sum_{i}^{N} Q_i c_{op}^i \right) \cdot \left( 1 - \%CO_2 \cdot \frac{MW_{CO_2}}{MW} - \%N_2 \cdot \frac{MW_{N_2}}{MW} \right) \quad (12)$$

wherein C02 and N2 components of the respective test gas are inserted and wherein MWco2 and MWN2 are the molar masses of C02 and N2.

**24.** Computer-implemented method according to claim 23, **characterised in that** HHVv is calculated by means of

$$HHV_v \quad \text{in } [MJ/m^3] = \rho_b \cdot HHV_m \quad \text{in } [MJ/kg] \qquad (13)$$

**25.** Computer-implemented method according to one of the preceding claims, **characterised in that** the method steps 1d.-1g. are carried out again if the input data from method step 1a. are re-entered.

**26.** Device for measuring the flow of gases with means for carrying out the method comprising a data storage device, an input device, a receiver unit, a flow meter and/or a computing unit, wherein the means are adapted to carry out the steps of the method according to one one of the claims 1-24.

**27.** Computer program comprising commands which, when the program is executed by a device for measuring the flow of gases according to claim 26, cause the device to carry out the method according to one of the claims 1-24.

**28.** Computer-readable medium containing instructions which, when carried out by a device for measuring the flow of gases according to claim 26, cause the device to carry out the method according to one of the claims 1-24.

**Revendications**

**1.** Procédé mis en œuvre par ordinateur pour mesurer le débit par ultrasons d'un gaz à un point de mesure à l'aide d'un débitmètre ultrasonique, sans connaître la composition du gaz/fluide, comprenant les étapes suivantes :

a. Création et enregistrement d'un ensemble de données relatives aux gaz d'essai, aux variables d'état $S_{test}$ des gaz d'essai et à leurs propriétés dans un dispositif de stockage de données,
b. Création d'un ensemble de données d'entrée à l'aide d'un dispositif d'entrée, les données d'entrée comprenant :

i. Conditions normales du point de mesure pour la température $T_b$ et la pression $P_b$,
ii. Point de fonctionnement au point de mesure pour la température $T_{OP}$ et la pression $P_{OP}$

iii. Plage de travail du point de mesure avec température ($T_{min}$, $T_{max}$) et pression ($P_{min}$, $P_{max}$),
iv. Au moins une composition gazeuse typique pour le point de mesure et sélection d'une composition gazeuse concrète d'un gaz de référence

c. Détermination de variables d'état $S_{ref}$ du gaz de référence à l'aide d'une unité de calcul, les variables d'état $S_{ref}$ étant au moins les suivantes : une compressibilité $z_{ref(T.P)}$ dans la plage de travail, une compressibilité $Z_{ref\_b(T\_b,P\_b)}$ dans la plage des conditions normales et/ou un pouvoir calorifique $HHV_{ref\_OP}$ et/ou une vitesse du son $C_{ref\_OP}$ au point de fonctionnement,
d. Détermination d'un sous-ensemble de gaz d'essai à l'aide de l'unité de calcul, les variables d'état $S_{test}$ des gaz d'essai étant comparées aux variables d'état $S_{ref}$ déterminées à l'étape c) et les gaz d'essai sélectionnés pour le sous-ensemble étant ceux qui se trouvent dans une plage de tolérance préalablement définie des variables d'état $S_{ref}$ et/ou qui ne se trouvent pas dans la plage de tolérance de la composition gazeuse indiquée au point b) iv.
e. Calcul des compressibilités $Z_{OP}$ de tous les gaz d'essai du sous-ensemble au point de fonctionnement et calcul des vitesses du son $C_{OP}$ de tous les gaz d'essai du sous-ensemble au point de fonctionnement à l'aide de l'unité de calcul et comparaison des compressibilités $Z_{OP}$ avec la compressibilité $Z_{ref\_OP}$, les différences $z_{OP\_diff}$ entre les compressibilités $Z_{OP}$ et la compressibilité $Z_{ref\_OP}$ étant approximées en fonction de $C_{OP}$,
f. Tri de toutes les valeurs fonctionnelles qui se situent en dehors d'une plage de tolérance de la fonction approximée.
g. Répétition des étapes e. et f. jusqu'à ce qu'un résidu soit inférieur à une valeur prédéfinie $R_{min}$ ou qu'un nombre minimum de gaz d'essai soit atteint, de sorte qu'il existe une sélection de gaz d'essai et qu'une approximation de la compressibilité z en fonction de la vitesse du son c et dans des conditions normales $Z_b$ soit effectuée,
h. Détermination des paramètres de régression estimés pour $z_{OP\_diff}$ et calcul des variables d'état complétes à l'aide de la compressibilité z déterminée dans g.
i. Réalisation d'une mesure du débit volumique de service à l'aide du débitmètre ultrasonique et conversion de la mesure du débit volumique de service en une mesure du débit volumique standard ou du débit massique à l'aide des variables d'état complétées déterminées.

**2.** Procédé mis en œuvre par ordinateur selon la revendication 1, **caractérisé en ce que** les gaz d'essai sont générés de manière aléatoire et/ou à partir de données de mesure.

**3.** Procédé mis en œuvre par ordinateur selon l'une des revendications précédentes, **caractérisé en ce que** les variables d'état complétées comprennent une densité, un pouvoir calorifique $HHV_v$ et/ou une masse molaire MW.

**4.** Procédé mis en œuvre par ordinateur selon la revendication 1 ou 2, **caractérisé en ce que** la compressibilité $z_{ref(T,P)}$ dans la plage de travail et la compressibilité $Z_{ref\_b(T\_b,P\_b)}$ dans la plage des conditions normales sont approximées.

**5.** Procédé mis en œuvre par ordinateur selon la revendication 4, **caractérisé en ce que** l'approximation est effectuée selon les fonctions suivantes (de préférence jusqu'à N=2) :

$$z_{Ref} = \sum_{i,j}^{N} K_{i,j} p^i T^j \qquad (1)$$

$$z_{Ref,b} = \sum_{i,j}^{N} K_{i,j} p_b^i T_b^j \qquad (2)$$

où $K_{i,j}$ sont les coefficients avec i et j de 0 à l'ordre N.

**6.** Procédé mis en œuvre par ordinateur selon la revendication 5, **caractérisé en ce que** les coefficients sont déterminés par une régression multiple du 2e degré selon P et T avec un critère d'écart minimal par rapport à la référence NEST/REFPROP/AGA/GERG.

**7.** Procédé mis en œuvre par ordinateur selon l'une des revendications précédentes, **caractérisé en ce que** le nombre de gaz d'essai se situe dans une plage prédéfinie.

8. Procédé mis en œuvre par ordinateur selon la revendication 7, **caractérisé en ce que** la plage prédéfinie comprend un nombre minimum de 3 et un nombre maximum de 100 gaz d'essai.

9. Procédé mis en œuvre par ordinateur selon l'une quelconque des revendications précédentes, **caractérisé en ce que**
la plage de tolérance T des variables d'état $S_{ref}$ comprend une plage de tolérance autour de la valeur de combustion du gaz de référence et/ou une plage de tolérance autour de la vitesse du son du gaz de référence.

10. Procédé mis en œuvre par ordinateur selon l'une quelconque des revendications précédentes, **caractérisé en ce que**
le calcul des compressibilités $Z_{OP}$ de tous les gaz d'essai du sous-ensemble au point de fonctionnement et le calcul de la vitesse due son $C_{OP}$ de tous les gaz d'essai du sous-ensemble au point de fonctionnement sont effectués à l'aide de l'unité de calcul à partir de la référence NIST/REFPROP/AGA/GERG.

11. Procédé mis en œuvre par ordinateur selon l'une quelconque des revendications précédentes, **caractérisé en ce que**
la différence $z_{OP\_diff}$ entre les compressibilités $Z_{OP}$ au point de fonctionnement et la compressibilité $Z_{ref\_OP}$ du gaz de référence peut être approximée à l'aide d'une analyse de régression en fonction du $C_{OP}$ en utilisant la fonction suivante, de préférence jusqu'à l'ordre N =2 :

$$z_{op} = \sum_{i}^{N} M_i c_{op}^i + z_{Ref} \qquad (3)$$

12. Procédé mis en œuvre par ordinateur selon l'une quelconque des revendications précédentes, **caractérisé en ce que**
lorsqu'un nombre minimal de la quantité partielle de gaz d'essai n'est pas atteint, l'ensemble de données saisi à l'étape 1 a. est complété par des propriétés de gaz synthétiques et les étapes 1d. à 1g. du procédé sont répétées.

13. Procédé mis en œuvre par ordinateur selon l'une quelconque des revendications précédentes, **caractérisé en ce que**,
lorsqu'un nombre maximal de sous-ensembles de gaz d'essai est dépassé, les étapes 1d à 1g sont répétées, la plage de tolérance T définie des variables d'état $S_{ref}$ et/ou la plage de tolérance de la valeur calorifique et/ou la plage de tolérance de la vitesse du son étant réduite(s).

14. Procédé mis en œuvre par ordinateur selon la revendication 1, **caractérisé en ce que**,
lorsqu'un nombre maximal de sous-ensembles de gaz d'essai est dépassé, les étapes 1d à 1g du procédé sont répétées, le résidu étant réduit.

15. Procédé mis en œuvre par ordinateur selon l'une quelconque des revendications précédentes, **caractérisé en ce que**
la compressibilité z est approximée en fonction de la vitesse du son et dans des conditions normales après l'étape g) du procédé à l'aide des équations suivantes :

$$z = \sum_{i}^{N} M_i (\delta \cdot c)^i + z_{Ref} \qquad (4)$$

$$z_b = \sum_{i}^{N} M_i (\delta_b \cdot c)^i + z_{Ref,b} \qquad (5)$$

où le facteur $\delta = d(t,p)$ dans l'équation 4 tient compte du rapport entre la vitesse du son $c_{ref}$ du gaz de référence dans des conditions de processus variables et la vitesse du son $C_{ref\_OP}$ du gaz de référence au point de fonctionnement, et où le facteur $\delta b = d(t_b,p_b)$ dans l'équation 5 tient compte du rapport entre la vitesse du son $C_{ref\_b}$ du gaz de référence dans des conditions normales et la vitesse du son $C_{ref\_OP}$ du gaz de référence au point de fonctionnement.

16. Procédé mis en œuvre par ordinateur selon l'une quelconque des revendications précédentes, **caractérisé en ce qu'** un coefficient adiabatique g(p,T) du gaz de référence est approximé à l'aide de l'équation suivante :

$$\gamma = \sum_{i,j}^{N} L_{i,j} p^i T^j \qquad (6)$$

17. Procédé mis en œuvre par ordinateur selon la revendication 16, **caractérisé en ce que** l'équation (6) est approximée de préférence jusqu'à l'ordre N=2, l'approximation étant effectuée par régression multiple du 2e degré selon P et T avec le critère d'écart minimal par rapport à la référence NIST/REFPROP/AGA/-GERG.

18. Procédé mis en œuvre par ordinateur selon la revendication 16 ou 17, **caractérisé en ce qu'**

une masse molaire $\Gamma$ est calculée à l'aide du coefficient adiabatiquey (p,T) selon l'équation suivante (7) :

$$\Gamma = RT \frac{\gamma}{c^2} \qquad (7)$$

où R est une constante universelle des gaz.

19. Procédé mis en œuvre par ordinateur selon la revendication 18, **caractérisé en ce qu'** une masse molaire MW de chaque gaz d'essai est déterminée à l'aide de l'équation suivante :

$$MW = \Gamma + f(c,p) \qquad (8)$$

20. Procédé mis en œuvre par ordinateur selon la revendication 19, **caractérisé en ce que**

f(c,p) est approximé à l'aide d'une approche selon l'équation 9 :

$$f(c,p) = \sum_{i}^{N} O_i p^i \cdot (\Gamma - M_{Ref}) \cdot \frac{2p_{op} - p}{p} \qquad (9)$$

de préférence jusqu'à l'ordre N=2, où $M_{Ref}$ est la masse molaire d'un composant principal du gaz de référence corrigée de la vitesse du son mesurée c.

21. Procédé mis en œuvre par ordinateur selon la revendication 19, **caractérisé en ce qu'** une densité de fonctionnement p et/ou une densité Pb dans des conditions normales sont calculées à l'aide de

$$\rho = \frac{p \cdot MW}{z \cdot RT} \qquad (10)$$

$$\rho_b = \frac{p_b \cdot MW}{z_b R T_b} \qquad (11)$$

**22.** Procédé mis en œuvre par ordinateur selon l'une quelconque des revendications précédentes, **caractérisé en ce que**,

pour chaque gaz d'essai de la sous-quantité déterminée de tous les gaz d'essai, un pouvoir calorifique supérieur $HHV_m$ (higher heating value), une masse molaire MW et une vitesse du son $C_{OP}$ au point de fonctionnement sont calculés à partir de la référence NIST/REFPROP/AGA/GERG, les valeurs du $HHV_m$ étant approximées en fonction de c.

**23.** Procédé mis en œuvre par ordinateur selon la revendication 22, **caractérisé en ce que**

l'approximation de $HHV_m$ s'effectue à l'aide de la fonction suivante :

$$HHV_m = \left( \sum_i^N Q_i c_{op}^i \right) \cdot \left( 1 - \%CO_2 \cdot \frac{MW_{CO_2}}{MW} - \%N_2 \cdot \frac{MW_{N_2}}{MW} \right) \quad (12)$$

où C02 et N2 sont les proportions du gaz d'essai respectif utilisé et où MWco2 et MWN2 sont les masses molaires du C02 et du N2.

**24.** Procédé mis en œuvre par ordinateur selon la revendication 23, **caractérisé en ce que**
$HHV_V$ est calculé à l'aide de

$$HHV_v \quad \text{in } [\text{MJ/m}^3] = \rho_b \cdot HHV_m \quad \text{in } [\text{MJ/kg}] \qquad (13)$$

**25.** Procédé mis en œuvre par ordinateur selon l'une quelconque des revendications précédentes, **caractérisé en ce que**
les étapes 1d à 1g du procédé sont à nouveau exécutées lorsque les données d'entrée de l'étape 1a du procédé sont à nouveau saisies.

**26.** Dispositif de mesure du débit de gaz à l'aide de moyens pour mettre en œuvre le procédé, comprenant un dispositif de stockage de données, un dispositif d'entrée, une unité de réception, un débitmètre et/ou une unité de calcul, les moyens étant adaptés pour exécuter les étapes du procédé selon l'une des revendications 1 à 24.

**27.** Programme informatique comprenant des instructions qui, lorsque le programme est exécuté par un dispositif de mesure du débit de gaz selon la revendication 26, amènent le dispositif à mettre en œuvre le procédé selon l'une des revendications 1 à 24.

**28.** Support lisible par ordinateur comprenant des instructions qui, lorsqu'elles sont exécutées par un dispositif de mesure du débit de gaz selon la revendication 26, amènent le dispositif à mettre en œuvre le procédé selon l'une des revendications 1 à 24.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2020112731 A
- DE 102015117468 A1
- CN 112345636 A
- WO 9518958 A1
- WO 2023114382 A1
- US 2003114992 A1
- EP 1063525 A2

**In der Beschreibung aufgeführte Nicht-Patentliteratur**

- **G. HOHEISEL**. FLUXUS Natural Gas Engine. *38th International North Sea Flow Measurement Workshop*